# EUROPEAN PATENT SPECIFICATION

(11) **EP 2 882 377 B1**
(45) Date of publication and mention of the grant of the patent: **23.11.2016**
(21) Application number: 14841325.5
(22) Date of filing: 01.10.2014
(51) Int. Cl.: A61B 17/88, A61B 17/72, A61B 17/86

(54) **TAPERED, CYLINDRICAL CRUCIFORM HAMMER TOE IMPLANT KIT**
KONISCHES, ZYLINDRISCHES UND KREUZFÖRMIGES HAMMERZEHIMPLANTAT-SET
KIT D'IMPLANT EFFILÉ, CYLINDRIQUE, CRUCIFORME POUR ORTEIL EN MARTEAU

(30) Priority: 11.10.2013 US 201314052046
(43) Date of publication of application: 17.06.2015
(60) Divisional application: 15169014.6
(73) Proprietor: Wright Medical Technology, Inc., Memphis, TN 38117 (US)
(72) Inventor: MCCORMICK, Daniel F., Memphis, TN 38119 (US)
(74) Representative: Hirsch & Associés
(86) International application number: PCT/US2014/058600
(87) International publication number: WO 2015/054001

(56) References cited:
- GB-A- 2 430 625
- US-A1- 2004 220 574
- US-A1- 2011 257 652
- US-A1- 2011 301 653
- US-A1- 2012 089 197
- US-A1- 2012 089 197
- US-A1- 2013 131 822
- US-A1- 2013 131 822
- US-B1- 6 458 134
- US-B1- 7 041 106
- US-B1- 7 041 106

## Description

### FIELD OF DISCLOSURE

The disclosed system and method relate to implants. More specifically, the disclosed system and method relate to installing an implant for treating hammer toe.

### BACKGROUND

Hammer toe is a deformity of the toe that affects the alignment of the bones adjacent to the proximal interphalangeal (PIP) joint. Hammer toe can cause pain and can lead to difficulty in walking or wearing shoes. A hammer toe can often result in an open sore or wound on the foot. In some instances, surgery can be required to correct the deformity by fusing one or both of the PIP and distal interphalangeal (DIP) joints.

The most common corrective surgery includes the placement of a pin or rod in the distal, middle, and proximal phalanxes of the foot to fuse the PIP and DIP joints. The pin or rod is cut at the tip of the toe, externally of the body. A plastic or polymeric ball is placed over the exposed end of the rod, which remains in the foot of the patient until the PIP and/or DIP joints are fused in approximately 6 to 12 weeks. This conventional treatment has several drawbacks such as preventing the patient from wearing closed toe shoes while the rod or pin is in place, and the plastic or polymeric ball can snag a bed sheet or other object due to it extending from the tip of the toe resulting in substantial pain for the patient.

Another conventional implant includes a pair of threaded members that are disposed within adjacent bones of a patient's foot. The implants are then coupled to one another through male-female connection mechanism, which is difficult to install in situ and has a tendency to separate.

Yet another conventional implant has body including an oval head and a pair of feet, which are initially compressed. The implant is formed from nitinol and is refrigerated until it is ready to be installed. The head and feet of the implant expand due to the rising temperature of the implant to provide an outward force on the surrounding bone when installed. However, the temperature sensitive material can result in the implant deploying or expanding prior to being installed, which requires a new implant to be used.

Documents US 2012/0089197 A1 and US 2011/0301653 A1 disclose implant kits, comprising:
a hammer toe implant comprising:
   - an elongated threaded portion; and
   - a blade portion extending from the elongated threaded portion, the blade portion having a substantially cylindrical cross-sectional geometry and a taper defined by a plurality of blades; and
an adapter configured for coupling to the hammer toe implant, said adapter including a sleeve having openings at each end and a bore longitudinally extending between said openings.

Accordingly, an improved implant for treating hammer toe is desirable.

### SUMMARY

The invention provides an implant kit as defined in claim 1. Preferred embodiments are defined in the dependent claims.

### BRIEF DESCRIPTION OF THE DRAWINGS

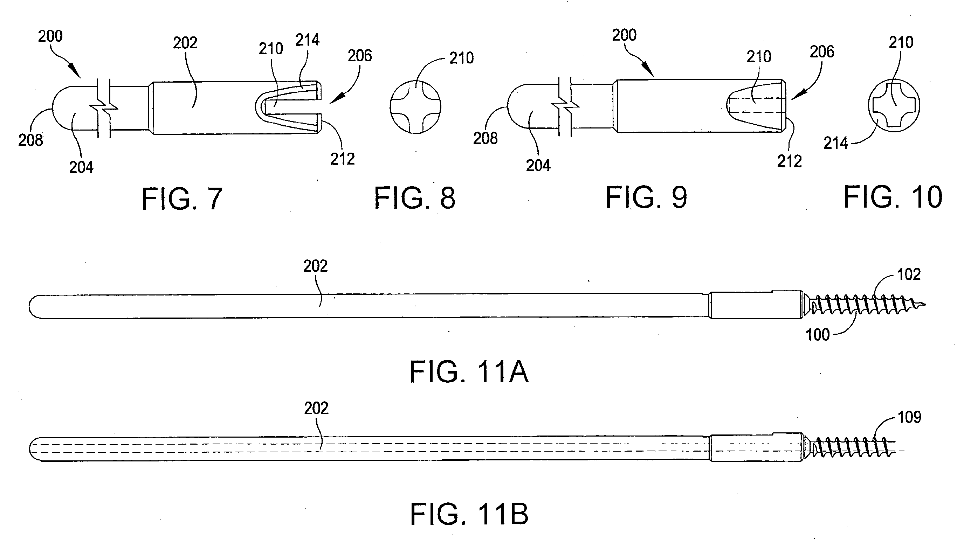

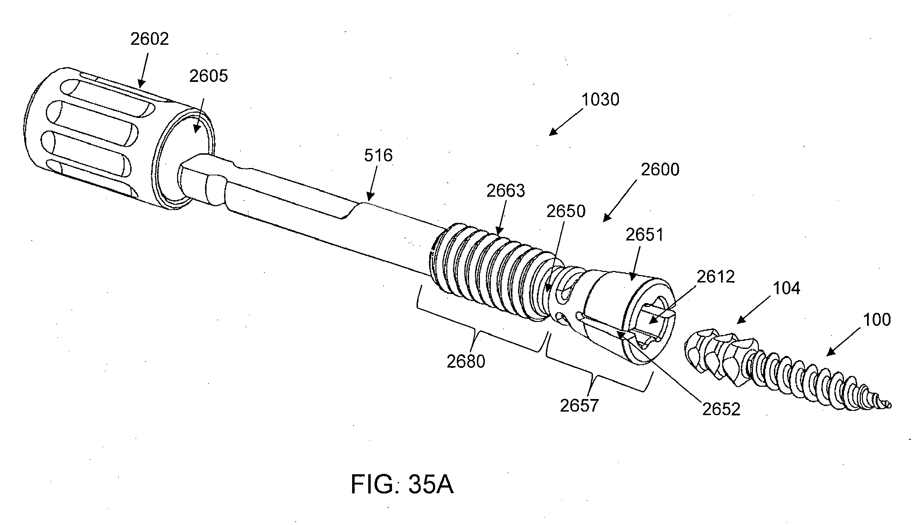

These and other features and advantages of the present invention will be more fully disclosed in, or rendered obvious by the following detailed description of the preferred embodiments of the invention, which are to be considered together with the accompanying drawings wherein like numbers refer to like parts, wherein only the implant kits shown in Figs. 35A-35F and described in paragraphs [0099] - [00102] are embodiments of the present invention, whereas the implant kits shown in Figs. 7-11B and 17-33C do not fall within the scope of the claims, and further wherein:
FIG. 1 is an isometric view of an improved hammer toe implant according to some embodiments;
FIG. 2 is a top side view of the hammer toe implant illustrated in FIG. 1;
FIG. 3 is a top side view of the blade portion of the hammer toe implant illustrated in FIG. 6;
FIG. 4 is a sectional view of the hammer toe implant taken along line 3-3 in FIG.
FIG. 5 is an end on view of the hammer toe implant taken along line 4-4 in FIG.
FIG. 6 is an isometric view of an improved, cannulated hammer toe implant according to some embodiments;
FIG. 7 is a side view of one example of a driving adapter for use with the hammer toe implants illustrated in FIGS. 1 and 6;
FIG. 8 is an end view of the driving adapter illustrated in FIG. 7;
FIG. 9 is a side view of another example of a driving adapter for use with the hammer toe implants illustrated in FIGS. 1 and 6;
FIG. 10 is an end view of the driving adapter illustrated in FIG. 9;
FIG. 11A is an assembly view of a hammer toe implant engaged by a driving adapter;
FIG. 11B is an assembly view of a cannulated hammer toe implant engaged by a cannulated driving adapter;
FIG. 12A illustrates the middle and proximal phalanxes of a foot being resected;
FIG. 12B illustrates the middle and proximal phalanxes of a foot being resected;
FIG. 13 illustrates a hammer toe implant being driven into a proximal phalanx;
FIG. 14 illustrates a middle phalanx being drilled or broached;
FIG. 15 illustrates a blade of a hammer toe implant extending from the proximal phalanx with the middle phalanx having been drilled or broached;
FIG. 16 illustrates a hammer toe implant installed in the middle and proximal phalanxes;
FIG. 17 illustrates another example of a driving assembly for installing an implant;
FIG. 18 illustrates a side view of the driving assembly illustrated in FIG. 17;
FIG. 19 is an isometric view of an adapter of the driving assembly illustrated in FIG. 17;
FIG. 20 is an end view of the adapter illustrated in FIG. 19;
FIG. 21 is a cross-sectional view of the adapter taken along line 21-21 in FIG. 20;
FIG. 22 is a cross-sectional view of the adapter taken along line 22-22 in FIG. 20;
FIG. 23 is a plan view of the driving rod of the driving assembly illustrated in FIG. 17;
FIG. 24 is a cross-sectional view of the driving rod taken along line 24-24 in FIG. 23;
FIG. 25 is a cross-sectional view of the fin of the driving rod taken along line 25-25 in FIG. 23;
FIG. 26 is a plan view of driving assembly illustrated in FIG. 17 without the o-ring;
FIG. 27 is a cross-sectional view of the handle taken along line 27-27 in FIG. 26;
FIG. 28A illustrates an implant coupled to the adapter of the driving assembly illustrated in FIG. 17;
FIG. 28B illustrates an implant coupled to the adapter of the driving assembly illustrated in FIG. 17;
FIG. 29 illustrates a hammer toe implant being driven into a proximal phalanx;
FIG. 30 illustrates an implant kit comprising a hammer toe implant preloaded in the adapter shown in FIGS. 19-22;
FIG. 31A is an isometric view of an implant kit according to some embodiments whose adapter has an implant receiving end configured to couple to an implant by an O-ring according to the adapter of FIGS. 19, 28A and 28B and having a driver shaft coupling end configured for coupling to the driver shaft by mating male and female threads;
FIG. 31B is an isometric view of an implant kit according to some embodiments whose adapter has an implant receiving end configured to couple to an implant by an O-ring according to the adapter of FIGS. 19, 28A and 28B and having a driver shaft coupling end configured for coupling to the driver shaft by a pair of opposing tabs;
FIG. 31C is a cross-sectional view of an adapter having a driver shaft coupling end illustrated in FIG. 31B and an implant receiving end according to some embodiments;
FIG. 31D is a cross-sectional view of an adapter having a driver shaft coupling end illustrated in FIG. 31A according to some embodiments;
FIG. 32A is an isometric view of an implant kit according to some embodiments whose adapter has an implant receiving end configured to couple to an implant by an O-ring according to the adapter of FIGS. 19, 28A and 28B and having a driver shaft coupling end configured for coupling to the driver shaft by an O-ring;
FIG. 32B is a side view of an adapter has an implant receiving end configured to couple to an implant by an O-ring according to the adapter of FIGS. 19, 28A and 28B and having a driver shaft coupling end configured for coupling to the driver shaft by an O-ring according to some embodiments;
FIG. 32C is a cross-sectional view of an implant kit whose adapter has a driver shaft coupling end for coupling to the driver shaft by an O-ring according to some embodiments;
FIG. 33A is a side view of an implant kit according to some embodiments whose adapter has an implant receiving end configured to couple to an implant by an O-ring according to the adapter of FIGS. 19, 28A and 28B and having a driver shaft coupling end configured for coupling to the driver shaft by an off-set clip;
FIG. 33B is an end perspective view of an adapter having a driver shaft coupling end configured for coupling to the driver shaft by an off-set clip according to some embodiments;
FIG. 33C is a cross-sectional view of an adapter having a driver shaft coupling end configured for coupling to the driver shaft by an off-set clip according to some embodiments;
FIG. 34A is an end perspective view of an adapter having a driver shaft coupling end configured for coupling to the driver shaft by a C-clip according to some embodiments;
FIG. 34B is an end view of an adapter having a driver shaft coupling end configured for coupling to the driver shaft by a C-clip according to some embodiments;
FIG. 34C is an end view of an adapter having a driver shaft coupling end configured for coupling to the driver shaft by a C-clip according to some embodiments;
FIG. 34D is cross-sectional view of an implant kit according to some embodiments having a driver shaft coupling end configured for coupling to the driver shaft by a C-clip;
FIG. 34E is a side view of a driver shaft configured for coupling to a driver shaft-coupling end of the adapter illustrated in FIG. 34D according to some embodiments;
FIG. 35A is an isometric view of some embodiments of an implant kit comprising an adapter that is configured for coupling to an hammer toe implant using a collet;
FIG. 35B is an isometric view of some embodiments of an implant kit comprising an adapter that is configured for coupling to an hammer toe implant using a collet and showing a hammer toe implant received in the adapter;
FIG. 35C is an isometric view of some embodiments of an implant kit comprising an adapter that is configured for coupling to an hammer toe implant using a collet;
FIG. 35D is a an end view of the implant kit illustrated in FIGS. 35B;
FIG. 35E is cross-sectional view of the implant kit taken along line 25-25 in FIG. 35D;
FIG. 35F is cross-sectional view of the implant kit taken along line 25-25 in FIG. 35D.

### DETAILED DESCRIPTION

This description of preferred embodiments is intended to be read in connection with the accompanying drawings, which are to be considered part of the entire written description. The drawing figures are not necessarily to scale and certain features of the invention can be shown exaggerated in scale or in somewhat schematic form in the interest of clarity and conciseness. In the description, relative terms such as "horizontal," "vertical," "up," "down," "top," and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral," and the like are to be interpreted relative to one another or relative to an axis of elongation, or an axis or center of rotation, as appropriate. Terms concerning attachments, coupling, and the like, such as "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship.

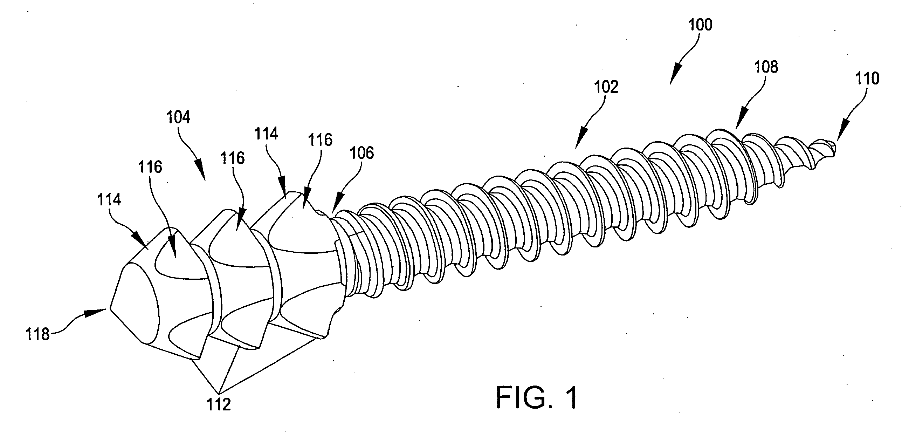

FIG. 1 illustrates one example of an improved implant 100 for treating hammer toe. As shown in FIG. 1, implant 100 includes a threaded portion 102 and a blade portion 104, which are connected together at an engagement portion 106. In some embodiments, implant 100 has a substantially linear geometry. In some embodiments, implant 100 has an overall length of approximately 19 mm (approximately 0.75 inches) (e.g. 18.9 - 19.1 mm (0.74-0.76 inches)). In some embodiments, blade portion 104 can be disposed at angle with respect to a longitudinal axis defined by the threaded portion 102. The angle can be between zero and 45 degrees, and more particularly between approximately five and fifteen degrees, although one skilled in the art will understand that implant 100 can have other dimensions and be provided in different sizes. For example, implant 100 can be provided in lengths of 16 mm and 22 mm, to name a few potential lengths.

In some embodiments, threaded portion 102 includes a plurality of threads 108 disposed along its entire length, which can be approximately 13 mm (approximately 0.5 inches) (e.g. 12.9 - 13.1 mm (0.49-0.51 inches)) although one skilled in the art will understand that threaded portion 102 can have other dimensions and be provided in different sizes. For example, threaded portion 102 can be provided in lengths of 10 mm and 15 mm, to name a few potential lengths. The tip 110 of threaded portion 102 can be pointed to facilitate the advancement of threads 108 into bone. Threads 108 can have a maximum outer diameter of approximately 2 mm (approximately 0.08 inches), although one skilled in the art will understand that thread portion 102 can have other dimensions and be configured to be received within a phalanx bone of a person. For example, threads can have an outer diameter of approximately 2.4 mm and 1.6 mm, to name a few potential possibilities.

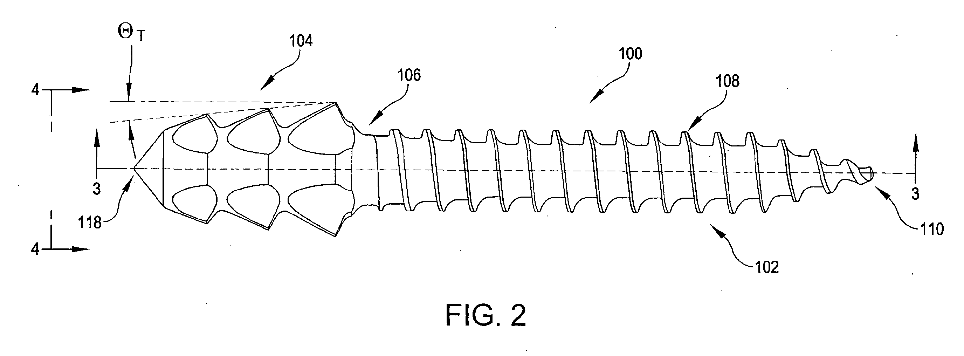

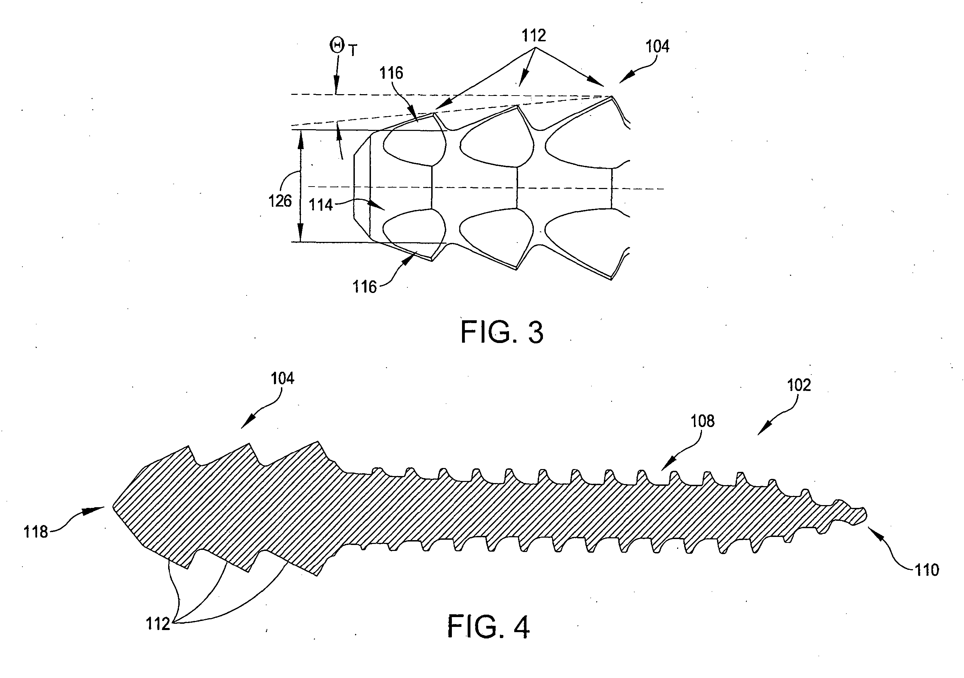

As shown in FIGS. 1-4 and 6, blade portion 104 can have a substantially cylindrical cross-sectional geometry. One skilled in the art will understand that blade portion 104 can have other cross-sectional geometries. In some embodiments, blade portion 104 can have a taper defined by a plurality of blades 112. For example, as best shown in FIGS. 2 and 3, the taper of blade portion 104 can be at an angle relative to the longitudinal axis defined by the elongated central portion of implant 100. In some embodiments, the taper is at an angle (Θ_{T}) between 1 and 10 degrees relative to the longitudinal axis defined by the elongated central portion of implant 100. For example, the taper can be at an angle (Θ_{T}) of approximately 5 degrees (e.g. 4.9 - 5.1 degrees) degrees relative to the longitudinal axis defined by the elongated central portion of implant 100. In some embodiments, blade portion 104 includes a taper along its diameter defined by the plurality of blades 112. In the illustrated embodiments of FIGS. 2, 3 and 5, the plurality of blades 112 include a first blade 112 having a first diameter disposed proximate the engagement portion 106 and a second blade 112 having a second diameter smaller than the first diameter disposed proximate a terminating end 118 of the blade portion 104. In some embodiments, the first diameter can be approximately 5 mm (approximately 0.20 inches) (e.g. 4.9 - 5.1 mm) (0.19 - 0.21 inches) and the second diameter can be approximately 4.5 mm (approximately 0.18 inches) (e.g. 4.4 - 4.6 mm) although one skilled in the art will understand that the plurality of blades 112 can have other diameters and other dimensions. For example, the first diameter can be provided as in lengths of 4 mm and 6 mm, to name a few potential diameters. The inventors have found that the tapered blade portion 104 permits each successive blade 112 of blade portion 104 to achieve interference with bone during insertion which enhances fixation of the blade portion 104 compared to a non-tapered blade portion 104. In the illustrated embodiment, the blades 112 of blade portion 104 include a valley 126 between blades 112 and the teeth portions 114 of each blade 112. In some embodiments, valley 126 of teeth portions 114 of each blade 112 is substantially the same. In other embodiments, the valleys 126 of teeth portions 114 vary as the respective diameters of the successive blades are tapered.

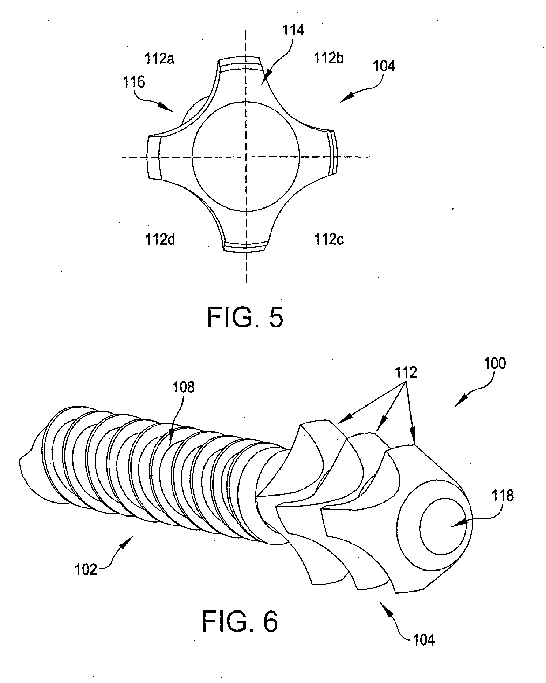

In some embodiments, the terminating end 118 of blade portion 104 is a point, although one skilled in the art will understand that blade portion 104 can have a terminating end of other dimensions, sizes and/or shapes. In the illustrated embodiment of FIGS. 3 and 6, the terminating end 118 of blade portion 104 is cannulated. In various embodiments (FIGS. 3 and 6), the blade portion 104 and threaded portion 102 of implant 100 are cannulated. In some embodiments, implant 100 (FIGS 3, 6, 11B) includes a groove 109 sized and configured to receive a k-wire, pin, or other surgical device or instrument that extends along the length of implant 100 in a direction that is parallel to a longitudinal length defined by implant 100. In some embodiments, the taper of blade portion 104 can be defined by the plurality of blades 112 having successively smaller diameters between a blade 112 disposed proximate engagement portion 106 and a blade 112 disposed proximate terminating end 118 of the blade portion 104.

In various embodiments, each blade 112 of the plurality of blades 112 of blade portion 104 include a plurality of grooved portions 116 and a plurality of teeth portions 114 to form a substantially cruciform cross-sectional geometry (FIG. 5). In the illustrated embodiment of FIG. 5, each blade 112 of blade portion 104 having a substantially cylindrical cross-section includes a plurality of substantially rounded grooved portions 116 formed along an axis parallel to a longitudinal axis of blade portion 104 and a plurality of teeth portions 114. As shown in FIGS. 1-5 and 6, blade portion 104 can have a substantially cylindrical cross-sectional geometry including a plurality of blades 112 having respective substantially cruciform cross-sectional geometries defined by a grooved portion 116 being disposed in each quadrant (112a-d) of each blade 112. In some embodiments, each blade 112 of blade portion 104 includes a pair of opposing grooved portions 116 (e.g. in quadrants 112b and 112d and in quadrants 112a and 112c respectively) to form a substantially cruciform cross-sectional geometry. As shown in FIG. 5, the grooved portions 116 of each pair of opposing grooved portions 116 (e.g. in quadrants 112a and 112c and 112b and 112d respectively) are substantially symmetrical. In the illustrated embodiment of FIG. 5, the grooved portions 116 disposed in each quadrant (112a-d) of each blade 112 are substantially symmetrical and the teeth portions 114 of each blade 112 are substantially symmetrical.

As shown more clearly in the illustrated embodiments of FIGS. 1,3,5 and 6, each blade 112 of blade portion 104 includes no flat surfaces. In some embodiments, a centerline of grooved portion 116 of each blade 112 of blade portion 104 is dimensioned such that it is tangent to respective diameters measured at the intersections of grooved portion 116 and the respective teeth portions 114. In the illustrated embodiments, grooved portions 116 are concave in shape and teeth portions 114 are convex in shape. In the illustrated embodiments, the respective surfaces of each blade 112 are rounded. In some embodiments, teeth portions 114 are serrated.

In some embodiments, engagement portion 106 can include a pair of protrusions extending from opposite sides of implant 100 and having rounded outer edges. In some embodiments, for example as shown in FIG. 2, the sides of the protrusions of engagement portion 106 can be substantially parallel with each other. In some embodiments, at least a portion of the implant 100 is cannulated (FIGS. 3, 6). The inventors have found that a cannulated implant 100 design can permit surgeons to stabilize joints (e.g. a metatarsal phalangeal joint (MPJ)) during a surgical procedure.

Implant 100 is configured to be installed using a driving adapter 200 such as the one illustrated in FIGS. 7-10. The driving adapter 200 has an elongated body 202 having a proximal end 204 and a distal end 206. Body 202 of driving adapter 200 can have a circular cross-sectional geometry, although one skilled in the art will understand that body 202 can have other cross-sectional geometries including, but not limited to, triangular, rectangular, pentagonal, and hexagonal to name a few.

Proximal end 204 can be substantially solid and have a rounded tip 208. In some embodiments, proximal end 204 and distal end 206 can be cannulated such as, for example, to receive a k-wire. Distal end 206 can define a slot 210 sized and configured to receive blade portion 104 of implant 100 therein. In some embodiments, slot 210 can have a cylindrical cross-sectional geometry and have a depth that is sufficient to receive the entire blade portion 104 of implant 100 such that distal edges 212 of slot 210 contact the protrusions of engagement portion 106. In some embodiments, slot 210 can have a cylindrical, cruciform cross-sectional geometry and have a depth that is sufficient to receive the entire blade portion 104 of implant 100 such that distal edges 212 of slot 210 contact the protrusions of engagement portion 106. However, one skilled in the art will understand that slot 210 can have other cross-sectional geometries and dimensions. Slot 210 can extend through side walls 214 of body 202 as shown in FIGS. 7 and 8, or side walls 214 can completely enclose slot 210 as shown in FIGS. 9 and 10.

If the driving adapter 200 is to be used with an implant 100 having a substantially linear lengthwise geometry such as the implant 100 illustrated in FIGS. 1-6, then slot 210 can extend in a direction that is substantially parallel to an axis defined by body 202 of driving adapter 200. If driving adapter 200 is to be used with an implant 100 having a blade portion 104 that extends at an angle with respect to an axis defined by elongated threaded portion 102, then slot 210 can extend from distal edges 212 at an angle with respect to an axis defined by the length of body 202 such that elongated threaded portion 102 of implant 100 is linearly aligned with body 202 of driving adapter 200 as shown in FIGS. 11A and 11B. For example, if blade portion 104 of implant 100 extends at a ten degree angle with respect to an axis defined by elongated threaded portion 102, then slot 210 of driving adapter 200 can extend at a ten degree angle with respect to a longitudinal axis defined by body 202 such that threaded portion 102 of implant 100 and body 202 of driving adapter 200 are substantially linearly aligned.

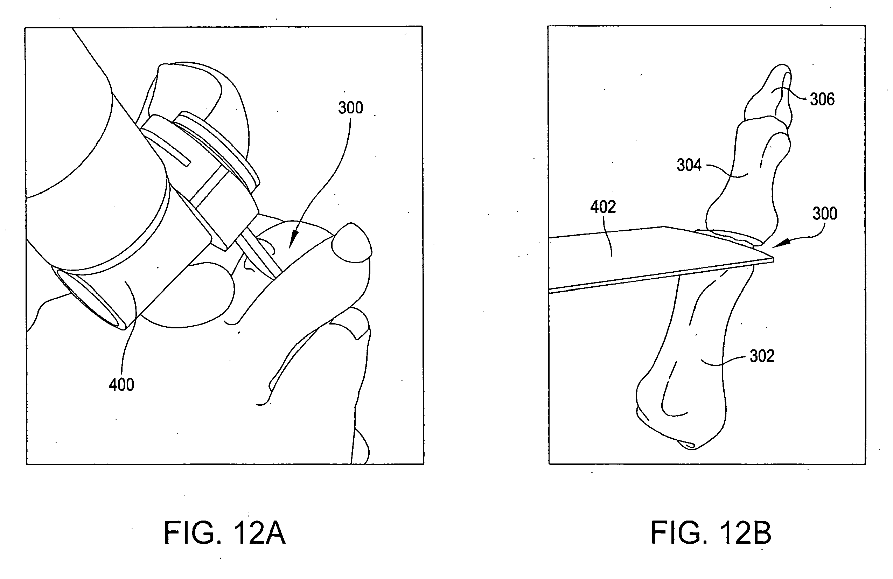

A method of installing implant 100 in the proximal interphalangeal joint (PIP) 300 is described with reference to FIGS. 12A-16. However, one skilled in the art will understand that the technique for installing the implant 100 can be applied to other joints such as, for example, the distal interphelangeal (DIP) joint between middle phalanx 304 and distal phalanx 306. As shown in FIGS. 12A and 12B, an incision is made to open the PIP joint 300 and a cutting tool 400 having a blade 402 can be used to resect adjacent faces of proximal phalanx 302 and middle phalanx 304. The resected surfaces of proximal phalanx 302 and middle phalanx 304 can be debrided as understood by one skilled in the art.

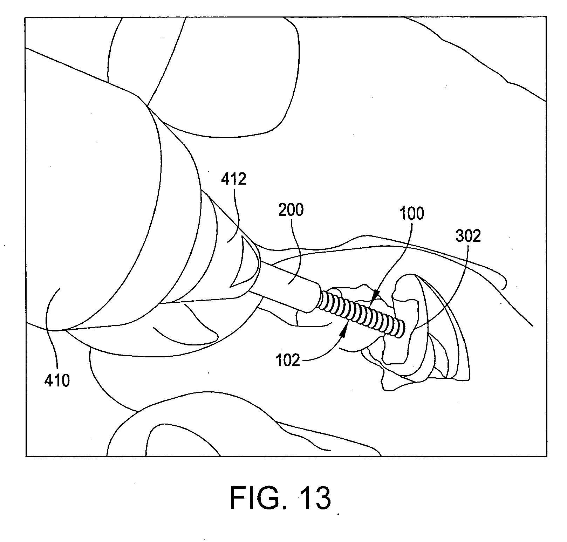

Blade portion 104 of implant 100 can be disposed within slot 210 of driving adapter 200 as shown in FIGS. 11A and 11B. In some embodiments, the body 202 of driving adapter 200 can be cannulated. In some embodiments, a k-wire, pin or other suitable surgical device can be inserted into the middle phalanx 304 and driven through distal phalanx and out of the end of the toe (not shown). A k-wire can be inserted such that a trailing end is disposed within middle phalanx 304 or otherwise positioned with respect to the joint such that cannulated implant 100 can be driven into proximal phalanx 302. In various embodiments, the body 202 of driving adapter 200 can be secured in a chuck 412 of a drill 410 or other driving instrument as shown in FIG. 13. Drill 410 or other driving instrument is used to drive the threaded portion 102 of implant 100 into the resected surface of proximal phalanx 302. With the threaded portion 102 of implant 100 disposed within proximal phalanx 302, driving adapter 200 can be disengaged from blade portion 104 of implant 100.

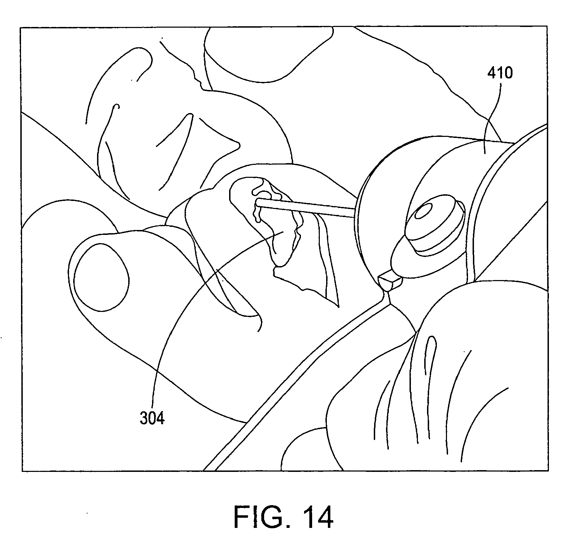

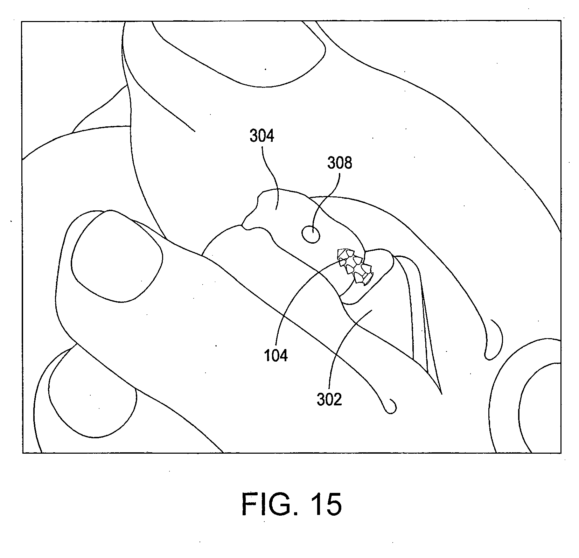

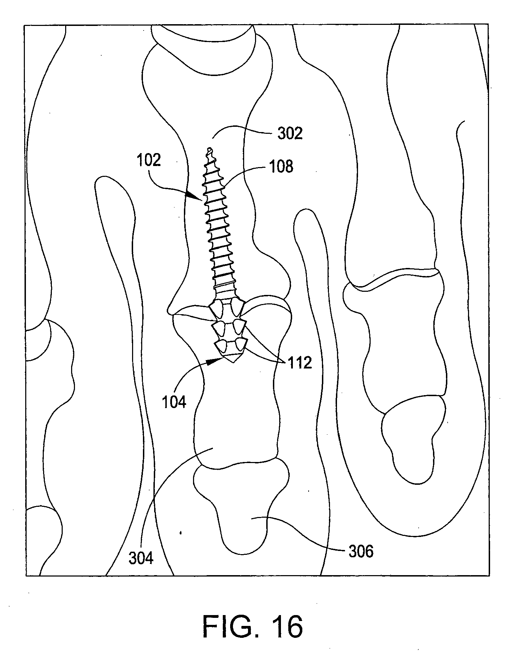

Middle phalanx 304 can be predrilled or broached using drill 410 to create a hole 308 as shown in FIGS. 14 and 15. The predrilled or broached middle phalanx 304 is then repositioned such that the predrilled hole or broach 308 aligns with the blade portion 104 of implant 100. In some embodiments, a dimension (e.g. diameter or width) of the predrilled hole or broach 308 is less than a dimension of blade portion 104 to permit a first blade 112 to achieve interference with the bone and enhance fixation of blade 104. For example, in some embodiments, "valley-to-valley" dimension of blade portion 104 (e.g. the diametrical dimension of blade portion 104 between blades 112). In some embodiments, a k-wire or other suitable surgical device is disposed within middle phalanx 304 can be aligned with groove 109 of cannulated implant 100 (FIGS. 3, 6, 11B) disposed within proximal phalanx 302. In various embodiments, the middle phalanx 304 can be then pressed into engagement with the blade portion 104 as shown in FIG. 16. Serrated teeth portions or edges 114 of blade portion 104 help to maintain the engagement between middle phalanx 304 and blade portion 104 of implant 100. In various embodiments, a k-wire or other suitable surgical device can be advanced into the joint, into and through middle phalanx 302, into the respective metatarsal and through cannulated implant 100. In various embodiments, the k-wire or other suitable surgical device can remain within the patient for a period of time, e.g. minutes, hours, days or months, and can then be removed to leave behind cannulated implant 100.

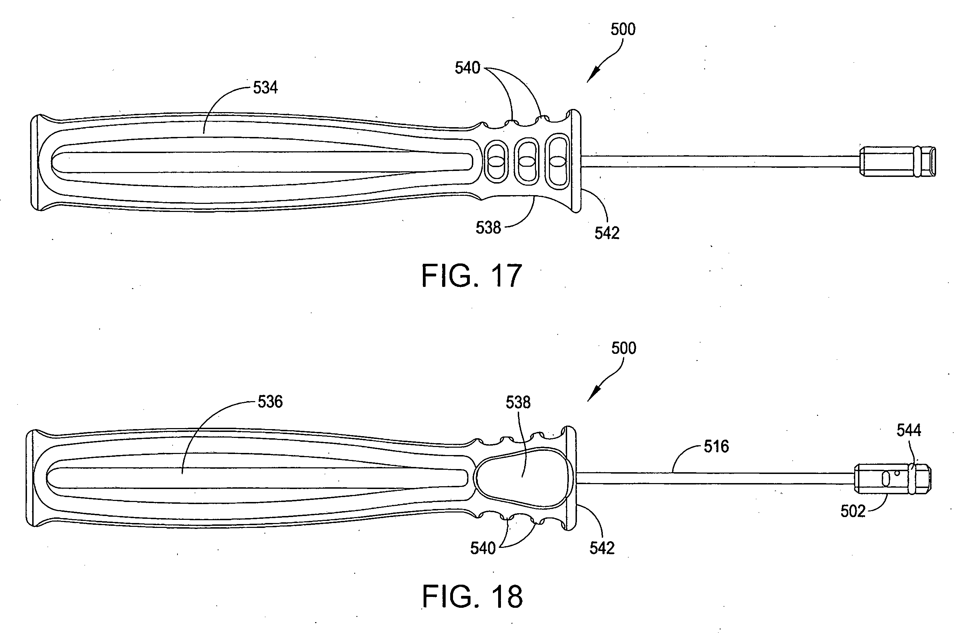

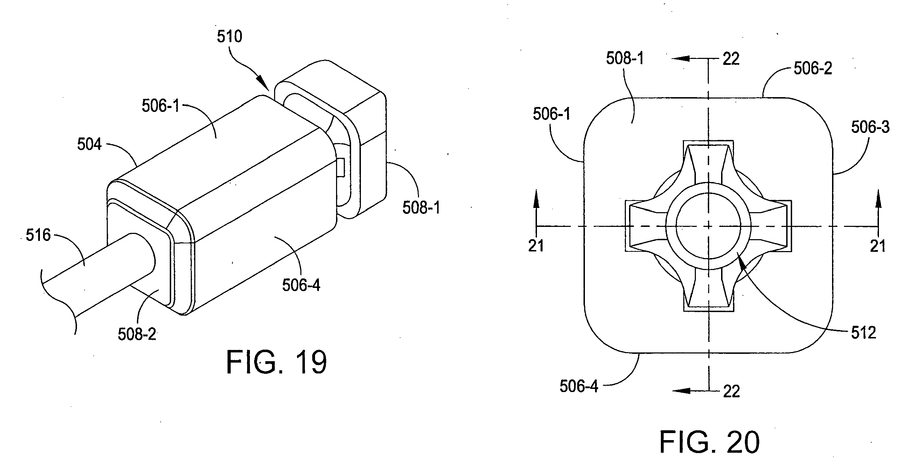

FIGS. 17-27 illustrate some embodiments of a driver assembly 500 for installing an implant into bone. As shown in FIGS. 17 and 18, driver assembly 500 includes an adapter 502 coupled to a driving rod 516 onto which a handle 534 is over-molded or otherwise coupled. Adapter 502 includes a body 504 with a substantially rectangular side profile comprising side walls 506-1, 506-2, 506-3, and 506-4 (collectively referred to as "side walls 506") and a pair of end walls 508-1, 508-2 (collectively referred to as "end walls 508") having a substantially square geometry as best seen in FIGS. 19-22.

Body 504 defines a recess 510 along the length of side walls 506. Recess 510 is dimensioned such that an o-ring 544 (FIGS. 17 and 18) can be received therein. Additionally, recess 510 is located along side walls 506 at a distance from end walls 508 such that recess 510 is aligned with a valley 126 of teeth portions 114 along the circumference of blade portion 104.

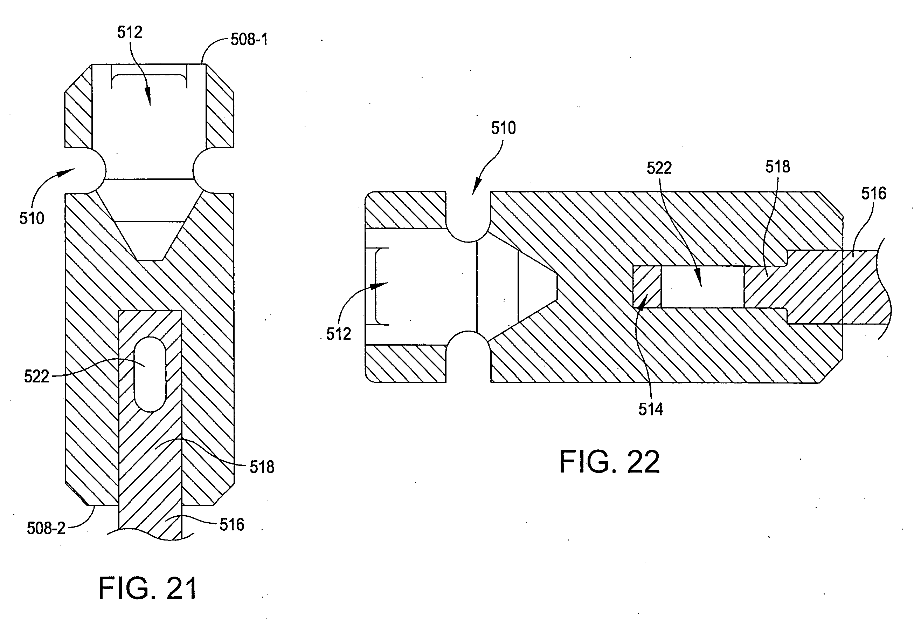

End wall 508-1 defines an aperture 512 (FIG. 20) having a geometry that complements the cross-sectional geometry of blade portion 104 of implant 100. For example, if implant 100 has a cylindrical, cruciform straight blade portion 104 as illustrated in FIG. 2, then aperture 512 can extend approximately parallel to the lengthwise direction of side walls 506 (FIGS 21-22). If the blade portion 104 of implant 100 is angled (not shown), then aperture 512 can extend from wall 508-1 at an angle relative to the plane defined by side wall 506-2 or 506-4 as will be understood by one skilled in the art. In some embodiments, aperture 512 has a depth that is greater than or equal to a length of blade portion 104 such that blade portion 104 can be received within body 504 and engagement portion 106 abuts end wall 508-1. Similarly, end wall 508-2 defines an aperture that is sized and configured to receive an end of elongated driving rod 516 therein.

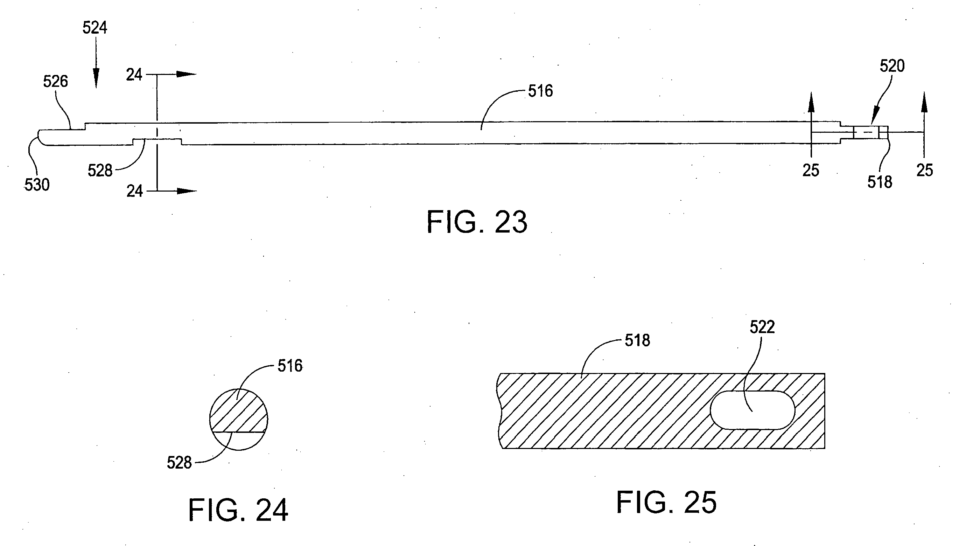

As best seen in FIGS. 23-25, driving rod 516 includes a fin 518 disposed at a first end 520. Fin 518 disposed at end 520 of driving rod 516 has a rectangular shape and is sized and configured to be received within aperture 512 of adapter 502. Fin 518 defines a slot 522, which is sized and configured to receive a pin (not shown) for cross-pinning driving rod 516 to adapter 502. In some embodiments, end 520 can have other cross-sectional geometries including, but not limited to, triangular, square, and pentagonal, to name a few possibilities, that are configured to be received within aperture 514. Adapter 502 can be over-molded onto the end of driving rod 516. However, one skilled in the art will understand that adapter 502 can be cross-pinned or otherwise coupled to driving rod 516.

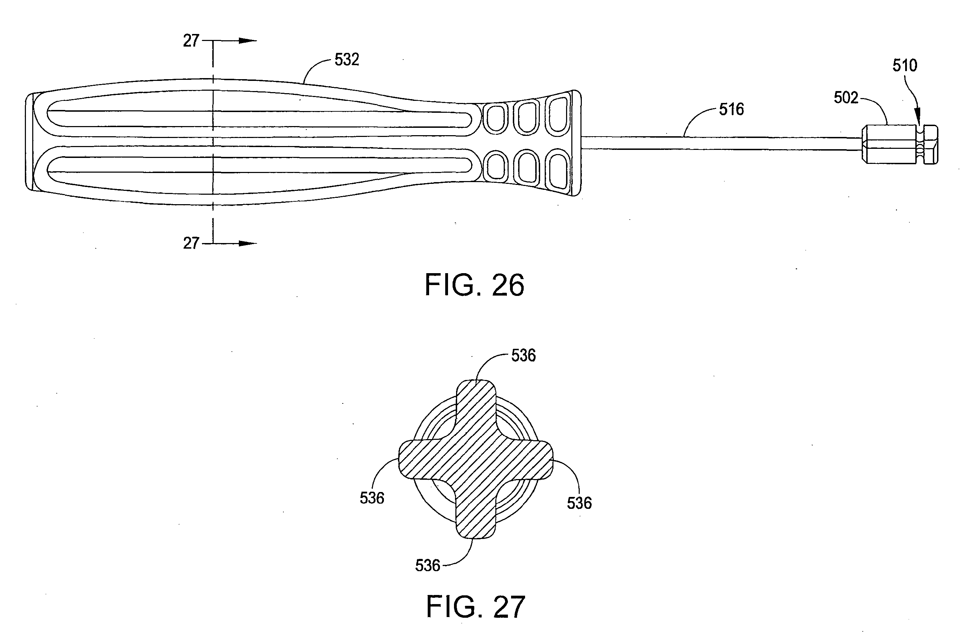

The opposite end 524 of driving rod 516 defines a pair of flats 526, 528, which are disposed on opposite sides of driving rod 516. As best seen in FIG. 23, flat 526 extends from tip 530 and is linearly spaced from flat 528, which is disposed at a greater distance from tip 530 than flat 526. However, one skilled in the art will understand that flats 526, 528 can be disposed at other positions along driving rod 516. Flats 526, 528 are configured to provide a contact surface for coupling to handle 532 (FIG. 26), which can be over-molded onto driving rod 516, such that rotation of handle 532 is translated to driving rod 516.

Turning now to FIGS. 26 and 27, handle 532 has an elongated body 534 that includes a plurality of ribs 536 that extend in a longitudinal direction along body 534 to provide a gripping surface for a user. As best seen in FIGS. 17 and 22, a smooth surface 538 interrupts circumferential ridges 540, which are disposed adjacent to proximal end 542 also for providing a gripping surface for a user.

Driver assembly 500 can be provided in a kit with a first adapter 502 for use with a straight implant 100 and a second adapter for use with an angled implant 100. A plurality of implants 100 of different sizes can also be provided in the kit. The kit can be used in an operation similar to the operation described above with respect to FIGS. 12A-16.

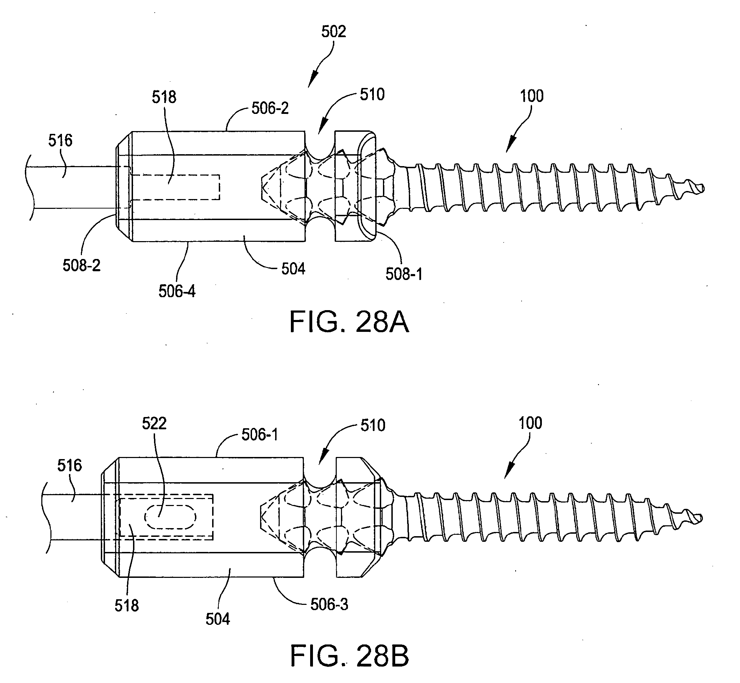

Blade portion 104 of implant 100 is disposed within aperture 512 of adapter 502 as shown in FIGS. 28A and 28B. With blade portion 104 disposed within aperture 512, an o-ring 544 (FIGS. 17 and 18) is placed in recess 510 defined by adapter 502 and within a valley 126 of serrated edges 112 along the top and bottom sides 114, 116 of blade portion 104. O-ring 544 secures implant 100 to adapter 502 such that implant does not move axially out of aperture 512.

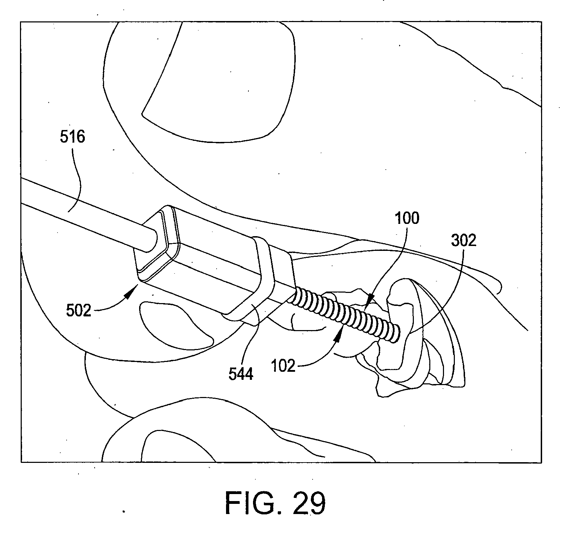

Once implant 100 is secured to adapter 502, the surgeon uses handle 534 to manually drive threaded portion 102 of implant 100 into the resected surface of proximal phalanx 302 as illustrated in FIG. 29. Implant 100 is driven into proximal phalanx 302 until engagement portion 106 abuts proximal phalanx 302. Implant 100 is decoupled from adapter 502 by axially pulling handle 534 away from implant 100 with sufficient force to flex o-ring 544 and separate adapter 502 from implant 100.

Middle phalanx 304 can be predrilled or broached using drill 410 to create a hole 308 as shown in FIGS. 14 and 15. The predrilled or broached middle phalanx 304 is then repositioned such that the predrilled hole or broach 308 aligns with the blade portion 104 of implant 100. The middle phalanx 304 is then pressed into engagement with the blade portion 104 as shown in FIG. 16. Serrated teeth portions 114 of blade portion 104 help to maintain the engagement between middle phalanx 304 and blade portion 104 of implant 100.

The implant described above can advantageously be installed through a small incision as described above. Additionally, the improved implant is completely disposed within a toe of a patient, which prevents the implant from being caught on bed sheets or other objects like the conventional pins.

According to an aspect of the present disclosure, the implant can be preloaded into an adapter and provided as an implant kit. Various embodiments of such an implant kit will be described below.

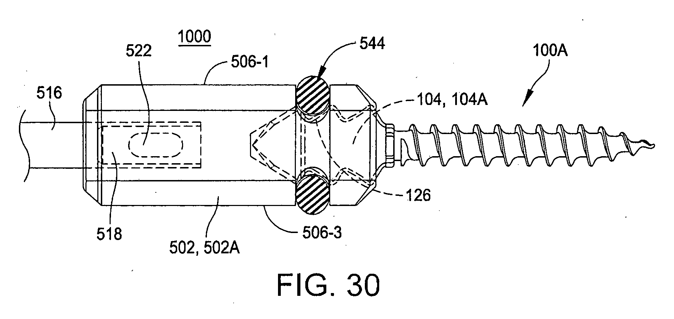

FIG. 30 is a view of the implant kit 1000 in which the implant 100, 100A is preloaded into the adapter 502. FIG. 30 is viewed from within the plane of FIGS. 28A and 28B so that the view shows the full circumference of the blade portion 104, 104A. In this view of FIG. 30, with the blade portion 104, 104A fully inserted into the adapter 502, 502A, an elastic O-ring 544 (also shown in FIGS. 17 and 18) placed in the groove 510 retains the implant 100, 100A in the adapter 502, 502A by preventing the implant from sliding out of the adapter. The cross-sections of the O-ring is shown in FIG. 30. The groove 510 is cut into the adapter with a sufficient depth so that when the O-ring 544 is placed therein the O-ring is positioned within the valley 126 between two adjacent teeth portions 114 about the circumference of the blade portion 104, 104A, as shown in FIG. 30. Because the O-ring 544 is elastic, one can push the blade portion 104, 104A of the implant into the adapter with sufficient force for one or more of the teeth portions 114 to push past the O-ring 544 when assembling the implant kit 1000. Once the implant kit 1000 is assembled, however, the O-ring 544 secures and retains the implant 100, 100A in the adapter 502 until one intentionally pulls off the adapter 502 after the implant is driven into a bone.

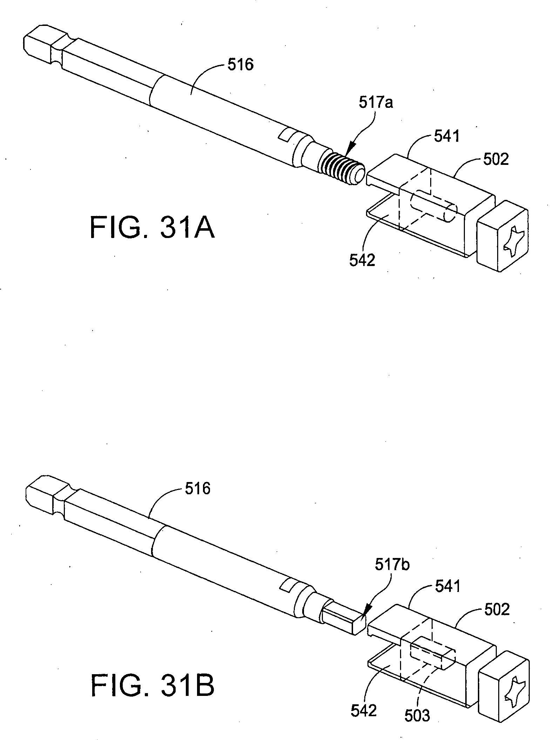

In use, the surgeon would attach the implant kit 1000 to the driver tool 500 to manually drive the threaded portion 102 of the implant 100, 100A into the resected surface of proximal phalanx 302 as illustrated in FIG. 29. The implant 100, 100A is driven into the proximal phalanx 302 until engagement portion 106 abuts the proximal phalanx 302. The implant 100, 100A is then decoupled from the adapter 502 by axially pulling the adapter 502 away from the implant 100, 100A with sufficient force to push the O-ring 544 outward and separate the adapter 502 from the implant 100, 100A. Referring to FIGS. 31A through 35F, various embodiments for removably coupling the implant kits disclosed above to a driver shaft 516 of a driver tool 500 will be described. FIGS. 31A- 31D are various views of some embodiments of an adapter such as the adapter 502 of FIGS. 28A, 28B, and 30 having a driver shaft coupling end configured for coupling to the adapter-engaging end 517a, 517b of the driver shaft. The driver shaft coupling end of the adapter 502 is provided with the longitudinally extending bore 514, configured for receiving the adapter-engaging end 517a, 517b, and a pair of opposing tabs 541, 542 extending longitudinally in the direction away from the implant engaging end. FIG. 31A shows a driver shaft 516 whose adapter-engaging end 517a is configured with screw threads. In this embodiment, the driver-engaging end of the adapter 502 is configured to threadably couple to the adapter-engaging end 517a of the driver shaft 502 and the tabs 541, 542 provide additional locking mechanism. FIG. 31B shows a driver shaft 516 whose adapter-engaging end 517b is configured with a magnetic tip. In this embodiment, the driver-engaging end of the adapter 502 is configured to magnetically couple to the adapter-engaging end 517b and the tabs 541, 542 provide additional locking mechanism. The adapter 502 would then be provided with a magnet or a piece of magnetic material 503 for magnetically coupling to the adapter-engaging end 517b.

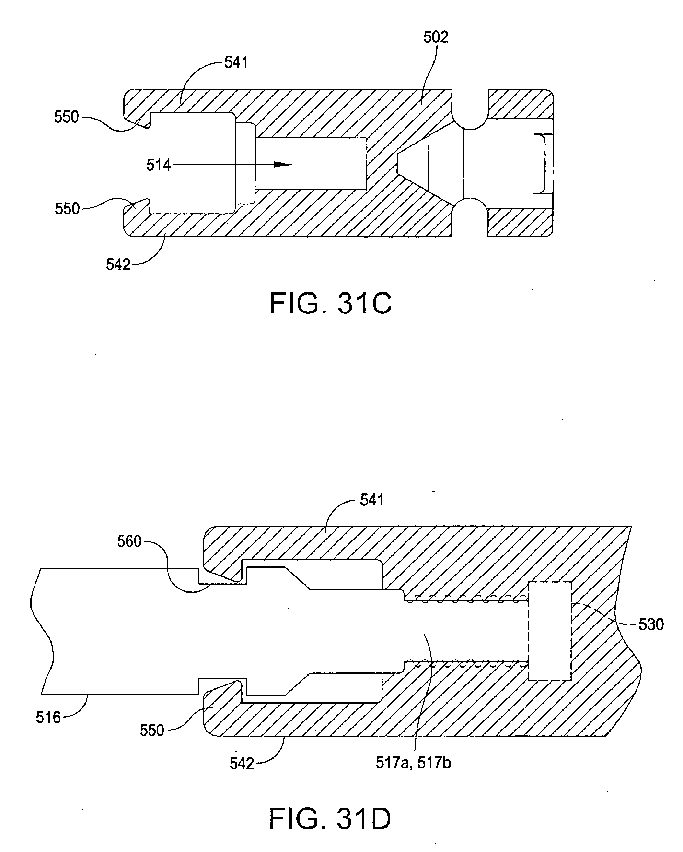

FIGS. 31C and 31D are cross-sectional views of the adapter 502 showing the driver-engaging end. FIG. 31C shows the profile of the tabs 541 and 542 and the bore 514 for receiving the adapter-engaging end 517 of the driver shaft. If the adapter 502 is intended for use with the driver shaft 516 of the embodiment shown in FIG 31A, the bore 514 is tapped with screw thread for threadably engaging the threaded adapter-engaging end 517a. If the adapter 502 is intended for use with the driver shaft 516 of the embodiment shown in FIG. 31B, the bore 514 is provided with a magnet 530 for engaging the magnetized tip of the adapter-engaging end 517b.

The tabs 541, 542 and the adapter-engaging end 517a, 517b are configured for further mechanical coupling. In the illustrated example, the tabs 541, 542 are provided with bumps 550 and the adapter-engaging end 517a, 517b of the driver shaft is provided with corresponding cutouts 560 for mating with the bumps 550.

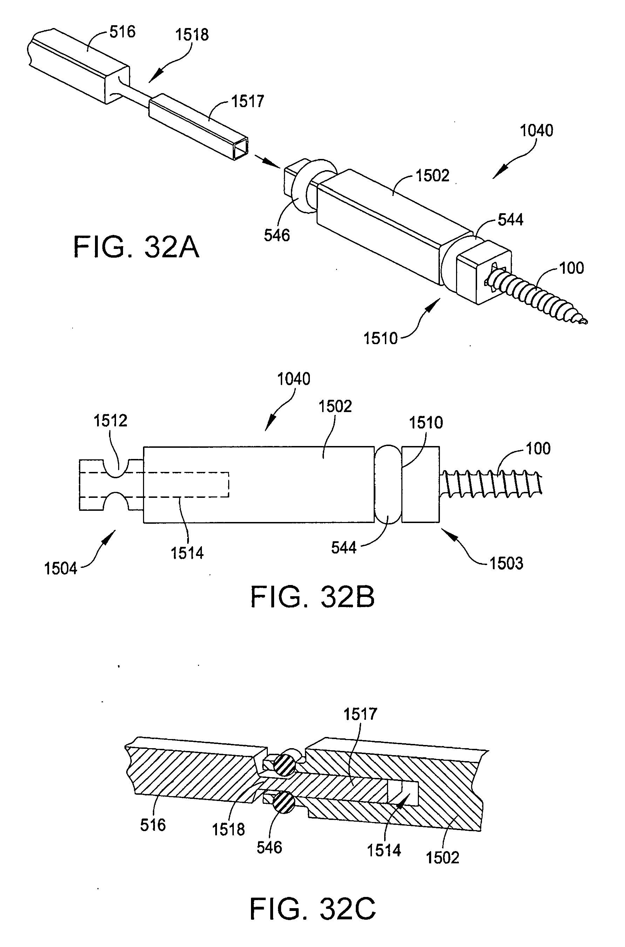

Shown in FIGS. 32A - 32C are various views of an implant kit 1040 comprising an adapter 1502 and an implant 100 according to some embodiments. The implant 100 is removably coupled to the adapter 1502 at the adapter's implant-receiving end 1503 by a first O-ring 544 in the same manner as with the adapter 502 shown in FIGS. 19, 28A, 28B and 30. The adapter 1502 has a circumferential groove 1510, in which the first O-ring 544 is provided, in the outer surface of the adapter in proximity to the implant-receiving end 1503. As with the adapter embodiment 502, the adapter 1502 comprises a slot provided in the implant-receiving end 1503 that receives the blade portion 104 of the implant 100. The adapter 1502 also has a driver shaft coupling end 1504 configured for removably coupling to the driver shaft 516 by a second O-ring 546. The driver shaft coupling end 1504 is provided with a longitudinally extending bore 1514 for receiving the adapter-engaging end 1517 of the driver shaft 516. The driver shaft coupling end 1504 is also provided with a second circumferential groove 1512 in which the second O-ring 546 is disposed. The adapter-engaging end 1517 has a cross-section that is larger than the inner diameter of the second O-ring 546 but has a turned down section 1518 that has a reduced cross-section for accommodating the second O-ring 546 when the adapter-engaging end 1517 is inserted into the bore 1514 as shown in FIG. 32C. When the adapter-engaging end 1517 is inserted into the bore 1514, the turned down section 1518 and the second circumferential groove 1512 align so that the second O-ring 546 rests in the turned down section 1518. The second O-ring 546 thus provides an interference with the adapter-engaging end 1517 to prevent the adapter 1502 and the driver shaft 516 from decoupling without exerting some force.

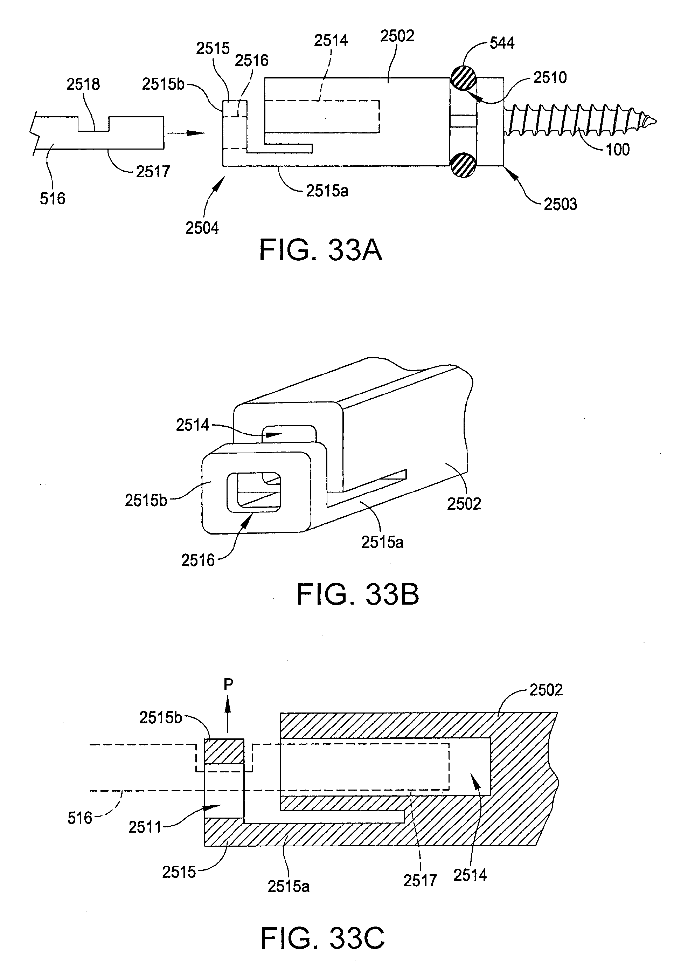

FIGS. 33A - 33C are various views of an adapter 2502 that can be used in an implant kit 1050 according to some embodiments of the present disclosure. The adapter 2502 has an implant receiving end 2503 configured to couple to an implant 100 by an O-ring 544 according to the adapter of FIGS. 19, 28A and 28B and a driver shaft coupling end 2504 configured for coupling to the driver shaft 516 by an off-set clip 2515. The driver shaft coupling end 2504 has a longitudinally extending bore 2514 for receiving an adapter-engaging end 2517 of the driver shaft 516. The off-set clip 2515 is cantilevered to the adapter having a cantilever portion 2515a connected to the adapter body and a locking portion 2515b extending orthogonal to the cantilever portion 2515a. The locking portion 2515b is provided with a through hole 2516 for the adapter-engaging end 2517 of the driver shaft 516. The through hole 2516 and the bore 2514 are off-set to enable the locking function. The adapter-engaging end 2517 is provided with a groove or a cutout 2518 on one side for removably engaging the off-set clip 2515. To insert the adapter-engaging end 2517 into the adapter, the user pushes the off-set clip 2515 in the direction shown by the arrow P in FIG. 33C, which is a longitudinal cross-sectional view of the adapter 2502. That will deflect the cantilever portion 2515a in the direction P and bring the through hole 2516 in linear alignment with the bore 2514 so that the adapter-engaging end 2517 can be inserted through the through hole 2516 and the bore 2514. Once the adapter-engaging end 2517 is fully inserted, the off-set clip 2515 is released to its normal off-set position as shown in FIG. 40C. The off-set position of the locking portion 2515b keeps the locking portion 2515b seated within the cutout 2518 keeping the driver shaft 516 coupled to the adapter 2502. The offset clip can be configured so that in the configuration shown in FIG. 33C, the locking portion 2515b maintains a force against the cutout 2518 in the direction opposite the arrow P. To remove the adapter 2502 from the adapter-engaging end 2517, the off-set clip 2515 is pushed in the direction of the arrow P shown in FIG. 33C bringing the through hole 2516 and the bore 2514 into longitudinal alignment and thus removing the interference between the locking portion 2515b and the cutout 2518. In some embodiments, the adapter-engaging end 2517 may simply be straight without the cutout 2518 structure. In that embodiment, the urging of the locking portion 2515b against the straight adapter-engaging end 2517 in the direction opposite the arrow P will provide sufficient frictional interference to keep the driver shaft 516 and the adapter 2502 coupled.

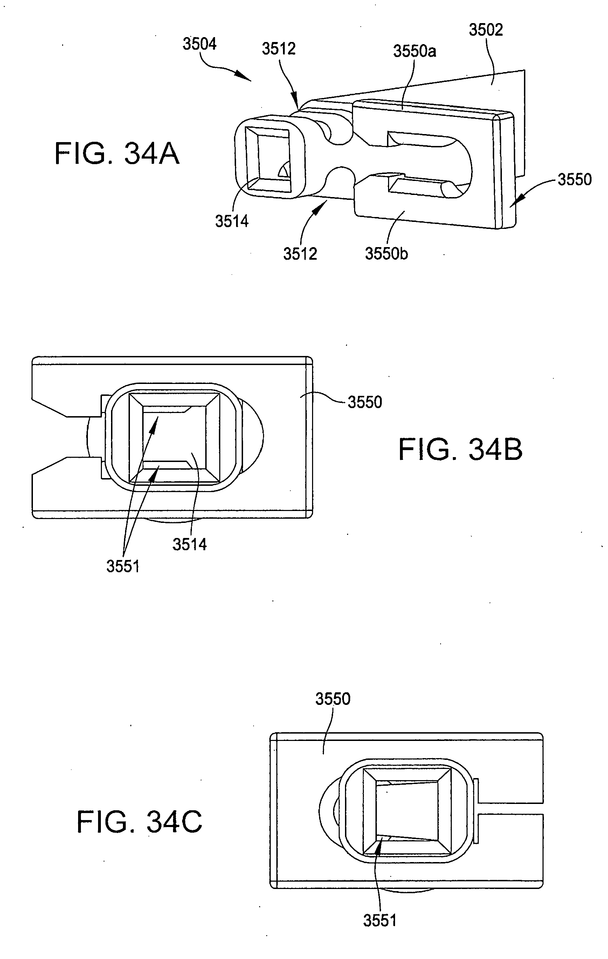

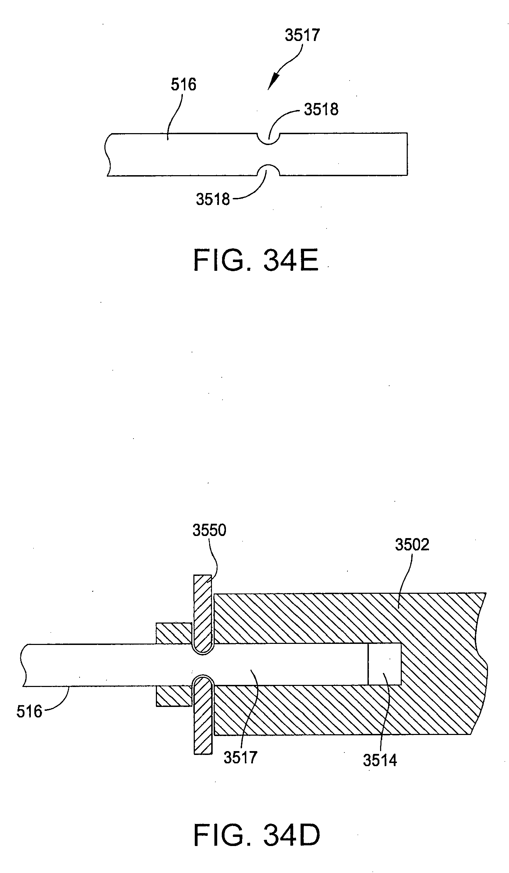

FIGS. 34A - 34E are various views of the driver shaft coupling end 3504 of an adapter 3502 that is configured for removably coupling to the implant 100 to form an implant kit according to some embodiments. The implant-receiving end of the adapter 3502 is configured to couple to the implant by an O-ring 544 according to the adapter of FIGS. 19, 28A and 28B. The driver shaft coupling end 3504 is configured to removably couple to the adapter-engaging end 3517 of the driver shaft 516 by a C-clip 3550. The C-clip 3550 is generally shaped like a letter C and has two prongs 3550a and 3550b joined at one end and open at the opposite end. The driver shaft coupling end 3504 of the adapter 3502 is provided with a bore 3514 for receiving the adapter-engaging end 3517. The driver shaft coupling end 3504 is further configured with a pair of slots 3512 for receiving the C-clip 3550 and oriented orthogonal to the longitudinal axis of the adapter 3502. FIG. 34B is an end view of the adapter assembly viewed from the driver shaft coupling end 3504 showing the C-clip 3550 clipped on to the adapter 3502 by sliding the two prongs 3550a, 3550b into the pair of slots 3512. The pair of slots 3512 are cut into the adapter 3502 sufficiently deep to overlap with the bore 3514 so that when the C-clip 3550 is clipped on to the adapter 3502, interference tabs 3551 on each of the two prongs 3550a, 3550b protrude into the bore 3514 as shown in FIG. 34B. When the adapter-engaging end 3517 of the driver shaft 516 is inserted into the bore 3514 and locked with the C-clip 3550 as shown in the longitudinal cross-sectional view of FIG. 34E, the interference tabs 3551 reside in the corresponding slots 3518 provided in the adapter-engaging end 3517 and prevent the adapter 3502 and the driver shaft 516 from decoupling. In this embodiment, the interference tabs 3551 are oriented substantially parallel to one another. In one preferred embodiment, the interference tabs 3551 can be oriented in a slant so that the interference tabs 3551 are tapered towards the open end of the C-clip 3550. The tapered interference tabs 3551 makes is easier to insert the C-clip 3550 over the adapter-engaging end 3517.

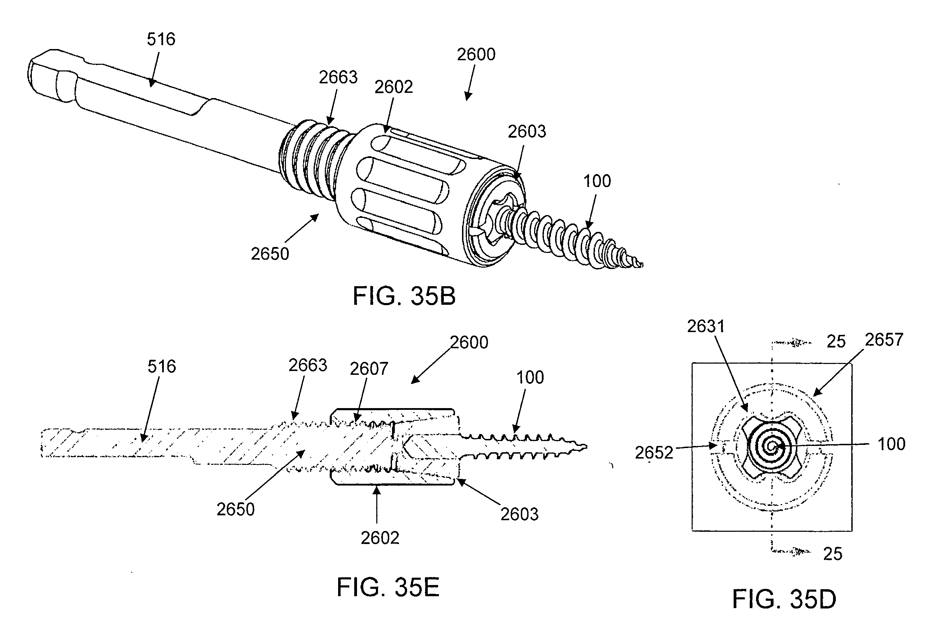

FIGS. 35A - 35F are various views of some embodiments of an implant kit 1030 comprising an adapter 2600 configured for coupling to a hammer toe implant 100 using a thread-biased collet 2650. The.adapter 2600 comprises a sleeve 2602 and the collet 2650. The sleeve 2602 has openings at each end and a bore 2615 longitudinally extending between the two openings. As shown in FIGS. 35A and 35B, sleeve 2602 can include a plurality of ribs that extend in a longitudinal direction along sleeve 2602 to provide a gripping surface for a user. The collet 2650 is received in the bore 2615. The sleeve 2602 has a first end 2605 that forms one of the openings.

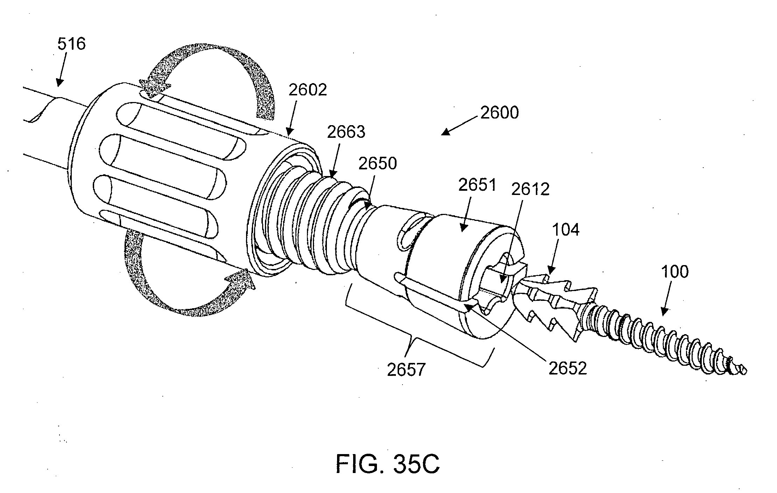

Referring to FIG. 35A, the collet 2650 is generally cylindrical in shape and comprises an implant receiving portion 2657 and a threaded portion 2660. The threaded portion 2660 is provided with screw threads 2663. The implant receiving portion 2657 has an implant-receiving opening 2612 for receiving the blade portion 104 of the implant 100. The implant-receiving opening 2612 is defined by collet segments 2651 which are defined by slots 2652 extending from the implant-receiving end towards the threaded portion 2660. This example of a collet has two collet segments 2651. The implant receiving portion 2657 is flared in its outer circumference so that the diameter of the receiving portion 2657 increases towards the implant-receiving end of the collet. FIG. 35B shows the collet 2650 with the implant 100 received in the slots 2652. FIG. 35C shows the collet with an indicated direction of rotation L to drive the sleeve 2602 onto the threaded portion 2660 to retain the implant 100 within implant receiving portion 2657. In various embodiments, the retraction and extension of the collet 2650 is enabled by turning the sleeve 2602 about a longitudinal axis relative to the collet 2650 thus engaging the screw threads 2607 and 2663. In some embodiments, sleeve 2602 is driven by hand in direction of rotation L to retain implant 100 within implant receiving portion 2657 pre-implantation and in an opposite direction of L to release implant 100 post-implantation.

FIG. 35D is an end view of the adapter 2600 illustrated in FIGS. 35A-35C and shows the implant 100 received in the implant receiving end 2657 and slots 2652. The implant-receiving opening 2612 of implant receiving portion 2657 has a geometry that complements the cross-sectional geometry of blade portion 104 of implant 100 and is defined by collet segments 2651 which are defined by slots 2652. For example, if implant 100 has a cylindrical, cruciform straight blade portion 104 as illustrated in FIG. 2 and FIG. 35A, then implant-receiving opening 2612 can extend approximately parallel to the lengthwise direction of collet 2650. If the blade portion 104 of implant 100 is angled (not shown), then implant-receiving opening 2612 can extend from end 2603 at an angle relative to the plane defined by collet 2650 as will be understood by one skilled in the art. In various embodiments, as shown in FIGS. 35A and 35D, collet segments 2651 of implant receiving end 2657 include radii features to complement radii features of the cylindrical, cruciform blade portion 104.

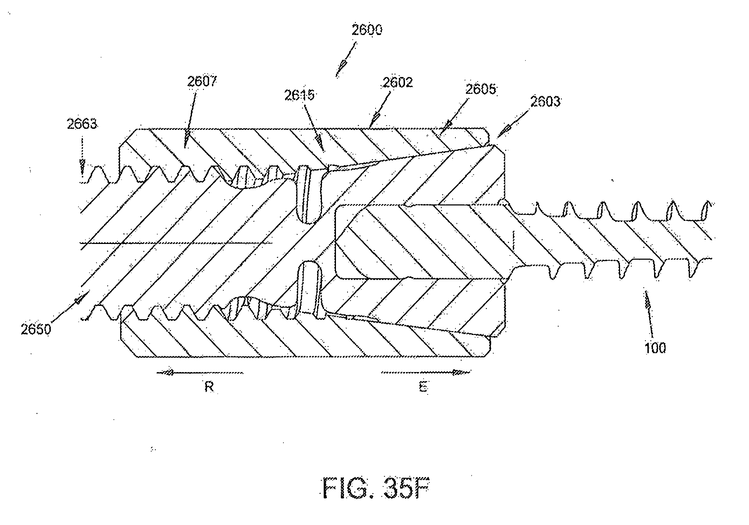

Referring now to FIGS. 35E and 35F, the bore 2615 has a screw threaded portion 2607 and a main portion 2605. The threaded portion 2607 is configured to threadably engage the threads 2663 of the collet 2650. The main portion 2605 has a sufficiently large diameter to accommodate a substantial portion of the implant receiving portion 2657 of the collet 2650 without imposing any mechanical interference. The main portion 2605 terminates at the first end 2603 where the opening formed therein has a diameter smaller than the maximum diameter of the flared implant receiving portion 2657. This configuration allows the collet segments 2651 to be constricted by the first end 2603 when the collet 2650 is retracted into the sleeve 2602 in the direction R shown in FIG. 35F and close in on the blade portion 104 of the implant 100, thus, retaining the implant. Conversely, the implant 100 can be released from the adapter 2600 by extending the collet 2650 outward from the sleeve 2602 in the direction E shown in FIG. 35F. In some embodiments, and as shown in FIG. 35F, sleeve 2602 includes an internal taper to interface with an external taper of the implant receiving portion 2657 of collet 2650.

Although the invention has been described in terms of exemplary embodiments, it is not limited thereto.

Some embodiments provide an implant including an elongated threaded portion and a blade portion extending from the elongated threaded portion. The blade portion includes a plurality of blades having respective substantially cruciform cross-sectional geometries defined by a grooved portion being disposed in each quadrant of each blade.

Although the invention has been described in terms of exemplary embodiments, it is not limited thereto.

## Claims

1. An implant kit (1030), comprising:
a hammer toe implant (100) comprising:
- an elongated threaded portion (102); and
- a blade portion (104) extending from the elongated threaded portion (102), the blade portion (104) having a substantially cylindrical cross-sectional geometry and a taper defined by a plurality of blades (112); and
an adapter (2600) configured for coupling to the hammer toe implant (100), said adapter (2600) including:
- a sleeve (2602) having openings at each end and a bore (2615) longitudinally extending between said openings at each end; and
- a thread-biased collet (2650) configured to be received in the bore (2615) and
comprising a threaded portion (2660) and an implant receiving portion (2657) having an implant-receiving opening (2612) for receiving the blade portion (104) of said hammer toe implant (100);
wherein the sleeve (2602) is configured to be rotated to drive the sleeve (2602) onto the threaded portion (2660) to retain the hammer toe implant (100) within the implant receiving portion (2657).

2. The implant kit (1030) of claim 1, wherein said implant-receiving opening (2612) is defined by collet segments (2651) which are defined by slots (2652) extending from the implant-receiving end toward the threaded portion (2660), said implant further comprising an engagement portion (106) disposed between the threaded portion (102) and the blade portion (104).

3. The implant kit (1030) of claim 2, wherein the plurality of blades (112) include a first blade (112) having a first diameter disposed proximate the engagement portion (106) and a second blade (112) having a second diameter smaller than the first diameter disposed proximate a terminating end (118) of the blade portion (104).

4. The implant kit (1030) of claim 2, wherein said implant-receiving opening (2612) of said implant receiving portion (2657) has a geometry that complements the cross-sectional geometry of said blade portion (104) and wherein each blade (112) of the plurality of blades (112) comprises a plurality of grooved portions (116) to form a substantially cruciform cross-sectional geometry.

5. The implant kit (1030) of claim 1, wherein each blade (112) of the plurality of blades (112) comprises a pair of opposing grooved portions (116) to form a substantially cruciform cross-sectional geometry.

6. The implant kit (1030) of claim 4, wherein said implant-receiving opening (2612) is defined by the slots (2652) of the collet segments (2651) and wherein the surfaces of each blade (112) are rounded.

7. The implant kit (1030) of claim 4, wherein said implant (100) has a cylindrical, cruciform straight blade portion (104) and said implant-receiving opening (2612) extends parallel to the lengthwise direction of said collet (2650).

8. The implant kit (1030) of claim 1, wherein the implant (100) is cannulated.

9. The implant kit (1030) of claim 4, wherein said blade portion (104) is angled and said implant-receiving opening (2612) extends from end (2603) at an angle relative to the plane defined by the collet (2650).

## Patentansprüche

1. Implantat-Set (1030), umfassend:
ein Hammerzehimplantat (100), umfassend:
- einen gestreckten Gewindeabschnitt (102); und
- einen Blattabschnitt (104), der vom gestreckten Gewindeabschnitt (102) verläuft, wobei der Blattabschnitt (104) eine im Wesentlichen zylindrische Querschnittgeometrie und eine Verjüngung aufweist, die durch mehrere Blätter (112) definiert ist; und
ein Passstück (2600), das zum Ankuppeln an das Hammerzehimplantat (100) konfiguriert ist, das Passstück (2600) enthaltend:
- eine Hülse (2602) mit Öffnungen an jedem Ende und einer Bohrung (2615), die längs zwischen den Öffnungen an jedem Ende verläuft; und
- einen gewindevorgespannten Ring (2650), der dazu konfiguriert ist, in der Bohrung (2615) aufgenommen zu sein, und einen Gewindeabschnitt (2660) und einen Implantataufnahmeabschnitt (2657) mit einer Implantataufnahmeöffnung (2612) zum Aufnehmen des Blattabschnitts (104) des Hammerzehimplantats (100) umfasst;
wobei die Hülse (2602) dazu konfiguriert ist, zum Treiben der Hülse (2602) auf den Gewindeabschnitt (2660) gedreht zu werden, um das Hammerzehimplantat (100) innerhalb des Implantataufnahmeabschnitts (2657) zu halten.

2. Implantat-Set (1030) nach Anspruch 1, wobei die Implantataufnahmeöffnung (2612) durch Ringsegmente (2651) definiert ist, die durch Schlitze (2652) definiert sind, welche vom Implantataufnahmeende zum Gewindeabschnitt (2660) hin verlaufen.

3. Implantat-Set (1030) nach Anspruch 2, wobei die mehreren Blätter (112) ein erstes Blatt (112) mit einem ersten Durchmesser, das proximal zum Eingriffsabschnitt (106) angeordnet ist, und ein zweites Blatt (112) mit einem zweiten Durchmesser, der kleiner als der erste Durchmesser ist, das proximal zu einem Abschlussende (118) des Blattabschnitts (104) angeordnet ist, enthalten.

4. Implantat-Set (1030) nach Anspruch 2, wobei die Implantataufnahmeöffnung (2612) des Implantataufnahmeabschnitts (2657) eine Geometrie aufweist, die die Querschnittsgeometrie des Blattabschnitts (104) komplementiert, und wobei jedes Blatt (112) der mehreren Blätter (112) mehrere genutete Abschnitte (116) zum Ausbilden einer im Wesentlichen kreuzförmigen Querschnittsgeometrie umfasst.

5. Implantat-Set (1030) nach Anspruch 1, wobei jedes Blatt (112) der mehreren Blätter (112) ein Paar gegenüberliegender genuteter Abschnitte (116) zum Ausbilden einer im Wesentlichen kreuzförmigen Querschnittsgeometrie umfasst.

6. Implantat-Set (1030) nach Anspruch 4, wobei die Implantataufnahmeöffnung (2612) durch die Schlitze (2652) der Ringsegmente (2651) definiert ist, und wobei die Oberflächen jedes Blatts (112) abgerundet sind.

7. Implantat-Set (1030) nach Anspruch 4, wobei das Implantat (100) einen zylindrischen, kreuzförmigen geraden Blattabschnitt (104) aufweist und die Implantataufnahmeöffnung (2612) parallel zur Längsrichtung des Rings (2650) verläuft.

8. Implantat-Set (1030) nach Anspruch 1, wobei das Implantat (100) kanüliert ist.

9. Implantat-Set (1030) nach Anspruch 4, wobei der Blattabschnitt (104) abgewinkelt ist und die Implantataufnahmeöffnung (2612) vom Ende (2603) in einem Winkel zur Ebene verläuft, die durch den Ring (2650) definiert ist.

## Revendications

1. Kit d'implant (1030) comprenant :
un implant de type marteau pour orteil (100) comprenant :
- une partie filetée allongée (102) ; et
- une partie de lame (104) s'étendant à partir de la partie filetée allongée (102), la partie de lame (104) ayant une géométrie transversale sensiblement cylindrique et une inclinaison définie par une pluralité de lames (112) ; et
un adaptateur (2600) configuré pour se coupler à l'implant de type marteau pour orteil (100), ledit adaptateur (2600) comprenant :
- un manchon (2602) ayant des ouvertures au niveau de chaque extrémité et un alésage (2615) s'étendant longitudinalement entre lesdites ouvertures au niveau de chaque extrémité ; et
- une pince de serrage sollicitée par filetage (2650) configurée pour être reçue dans l'alésage (2615) et comprenant une partie filetée (2660) et une partie de réception d'implant (2657) ayant une ouverture de réception d'implant (2612) pour recevoir la partie de lame (104) dudit implant de type marteau pour orteil (100) ;
dans lequel le manchon (2602) est configuré pour être entraîné en rotation pour entraîner le manchon (2602) sur la partie filetée (2660) afin de retenir l'implant de type marteau pour orteil (100) à l'intérieur de la partie de réception d'implant (2657).

2. Kit d'implant (1030) selon la revendication 1, dans lequel ladite ouverture de réception d'implant (2612) est définie par des segments de pince de serrage (2651) qui sont définis par des encoches (2652) s'étendant à partir de l'extrémité de réception d'implant vers la partie filetée (2660),
ledit implant comprenant en outre une partie de mise en prise (106) disposée entre la partie filetée (102) et la partie de lame (104).

3. Kit d'implant (1030) selon la revendication 2, dans lequel la pluralité de lames (112) comprend une première lame (112) ayant un premier diamètre disposé à proximité de la partie de mise en prise (106) et une seconde lame (112) ayant un second diamètre inférieur au premier diamètre disposée à proximité de l'extrémité terminale (118) de la partie de lame (104).

4. Kit d'implant (1030) selon la revendication 2, dans lequel ladite ouverture de réception d'implant (2612) de ladite partie de réception d'implant (2657) a une géométrie qui complète la géométrie transversale de ladite partie de lame (104) et dans lequel chaque lame (112) de la pluralité de lames (112) comprend une pluralité de parties rainurées (116) afin de former une géométrie transversale sensiblement cruciforme.

5. Kit d'implant (1030) selon la revendication 1, dans lequel chaque lame (112) de la pluralité de lames (112) comprend une paire de parties rainurées opposées (116) afin de former une géométrie transversale sensiblement cruciforme.

6. Kit d'implant (1030) selon la revendication 4, dans lequel ladite ouverture de réception d'implant (2612) est définie par les encoches (2652) des segments de pince de serrage (2651) et dans lequel les surfaces de chaque lame (112) sont arrondies.

7. Kit d'implant (1030) selon la revendication 4, dans lequel ledit implant (100) a une partie de lame cylindrique, cruciforme, droite (104) et ladite ouverture de réception d'implant (2612) s'étend parallèlement au sens de la longueur de ladite pince de serrage (2650).

8. Kit d'implant (1030) selon la revendication 1, dans lequel l'implant (100) est tubulaire.

9. Kit d'implant (1030) selon la revendication 4, dans lequel ladite partie de lame (104) est coudée et ladite ouverture de réception d'implant (2612) s'étend partir de l'extrémité (2603) à un angle par rapport au plan défini par la pince de serrage (2650).
